# FASCICULE DE BREVET EUROPEEN

(11) **EP 0 555 914 B1**
(45) Date de publication et mention de la délivrance du brevet: **22.11.1995**
(21) Numéro de dépôt: 93200292.6
(22) Date de dépôt: 04.02.1993
(51) Int. Cl.: C07C 17/42, C09K 5/04, C09K 3/30

(54) **Procédé pour la stabilisation de compositions comprenant des hydrofluoroalcanes et compositions stabilisées comprenant des hydrofluoroalcanes**
Verfahren zur Stabilisierung von Fluorkohlenwasserstoffen enthaltenden Gemischen und stabilisierte Gemische, die Fluorkohlenwasserstoffe enthalten
Process for the stabilization of compositions containing hydrofluoroalkanes and stabilized compositions containing hydrofluoroalkanes

(30) Priorité: 14.02.1992 BE 9200161
(43) Date de publication de la demande: 18.08.1993
(73) Titulaire: SOLVAY (Société Anonyme), B-1050 Bruxelles (BE)
(72) Inventeur: Barthelemy, Pierre, B-1370 Jodoigne (BE); Zipfel, Lothar, W-3014 Laatzen 3 (DE); Benecke, Thomas, W-3000 Hannover 91 (DE)
(74) Mandataire: Jacques, Philippe

(56) Documents cités:
- FR-A- 1 375 697
- CHEMICAL ABSTRACTS, vol. 81, no. 19, 11 Novembre 1974, Columbus, Ohio, US; abstract no. 119905m, TAKESHI KAWAMOTO ET AL. 'Stabilization of chlorinated hydrocarbons' page 473 ;colonne 2 ;
- D1 : EP-A-503441 (Solvay Fluor und Derivative)

## Description

La présente invention concerne un procédé pour la stabilisation de compositions comprenant des hydrofluoroalcanes ainsi que des compositions stabilisées comprenant des hydrofluoroalcanes.

Les hydrocarbures chlorofluorés entièrement halogénés (CFC) suspectés d'avoir un effet néfaste sur la couche d'ozone peuvent être substitués dans bon nombre d'applications, telles que par exemple l'utilisation comme agent gonflant pour la préparation de mousses, code fluide caloporteur ou comme propulseur, par des hydrocarbures fluorés partiellement halogénés, encore appelés hydrofluoroalcanes (HFA).

Les HFA sont cependant généralement moins stables que les CFC, de sorte que leur utilisation dans les domaines mentionnés ci-dessus pose encore un problème de stabilité.

La demande de brevet EP-503441, publiée le 16 septembre 1992, décrit des compositions comprenant du dichlorotrifluoroéthane, stabilisées par addition d'au moins un sel de lithium ou de métal alcalino-terreux.

La présente invention a pour objet de procurer de nouveaux stabilisants efficaces des hydrofluoroalcanes au sein des compositions qui les comprennent.

L'invention concerne un procédé pour la stabilisation de compositions comprenant au moins un hydrofluoroalcane autre que le dichlorotrifluoroéthane, caractérisé en ce qu'on y ajoute au moins un sel d'au moins un métal choisi parmi le lithium et/ou les métaux alcalino-terreux en quantité suffisante pour stabiliser l'hydrofluoroalcane compris dans la composition.

Par hydrofluoroalcanes, on entend généralement désigner les hydrocarbures aliphatiques halogénés saturés comprenant au moins un atome de fluor et au moins un atome d'hydrogène. Ces hydrofluoroalcanes peuvent ou non comprendre en outre un ou plusieurs atomes de chlore. De préférence, ils en comprennent au moins un. Les hydrofluoroalcanes tels que définis comprennent généralement de 1 à 3 atomes de carbone. A titre d'exemple de tels hydrofluoroalcanes, on peut mentionner le 1,1-dichloro-1-fluoroéthane.

Le procédé selon l'invention peut être mis en oeuvre pour la stabilisation de compositions comprenant un ou plusieurs hydrofluoroalcanes. Il peut par ailleurs être mis en oeuvre pour la stabilisation de compositions essentiellement constituées d'un ou de plusieurs hydrofluoroalcanes.

Parmi les métaux alcalino-terreux, on met en particulier en oeuvre le magnésium, le calcium, le strontium et le baryum. On choisit plus particulièrement le magnésium, le calcium ou le strontium et de préférence le magnésium ou le calcium. D'excellents résultats ont été obtenus avec le calcium.

Les sels mis en oeuvre peuvent être des sels organiques ou inorganiques. Parmi les sels inorganiques, on peut en particulier utiliser les nitrates ou les halogénures. Parmi ces derniers, on retient plus particulièrement les fluorures, les bromures ou les chlorures. D'excellents résultats ont été obtenus avec les chlorures.

Les sels stabilisants selon l'invention peuvent être utilisés sous forme anhydre ou hydratée. Ils sont de préférence mis en oeuvre à l'état solide.

Les sels stabilisants selon l'invention peuvent être utilisés à des doses variables. Ils sont généralement utilisés à raison de 0,05 % en poids au moins par rapport au poids total d'hydrofluoroalcanes compris dans la composition. De préférence, on en utilise au moins 0,1 %. Par ailleurs, on ne dépasse habituellement pas 5 % en poids de sel d'au moins un métal choisi parmi le lithium et/ou les métaux alcalino-terreux par rapport au poids total d'hydrofluoroalcanes compris dans la composition. De préférence, on ne dépasse pas 2 %.

D'autres agents stabilisants et/ou d'autres additifs peuvent également être ajoutés aux hydrofluoroalcanes dans les compositions stabilisées selon l'invention.

La présente invention concerne également des compositions comprenant au moins un hydrofluoroalcane autre que le dichlorotrifluoroéthane, caractérisées en ce qu'elles comprennent au moins un sel d'au moins un métal choisi parmi le lithium et/ou les métaux alcalino-terreux en quantité suffisante pour stabiliser l'hydrofluoroalcane compris dans la composition.

La nature des sels stabilisants et les quantités dans lesquelles ceux-ci sont avantageusement mis en oeuvre, de même que la nature des compositions comprenant l'hydrofluoroalcane sont celles décrites ci-dessus dans le cadre du procédé selon l'invention.

Les compositions selon l'invention peuvent avantageusement être mises en oeuvre code agents gonflants, code fluides caloporteurs, comme propulseurs ou encore comme solvants.

Les compositions selon l'invention peuvent en particulier être constituées de prémélanges, comprenant des hydrofluoroalcanes comme agents gonflants, destinés à la préparation de mousses, telles que des mousses de polyuréthane. Ces compositions sont plus particulièrement constituées de prémélanges à base de polyols, comprenant des hydrofluoroalcanes comme agents gonflants, destinés à la fabrication de mousses de polyuréthane. D'excellents résultats ont été obtenus avec des compositions constituées de prémélanges à base de polyols, comprenant du 1,1-dichloro-1-fluoroéthane comme agent gonflant, destinés à la fabrication de mousses de polyuréthane. De telles compositions peuvent également comprendre un ou plusieurs autres agents gonflants. Elles se sont avérées particulièrement stables en présence de chlorure de calcium, ne donnant lieu en particulier qu'à une formation très réduite de 1-chloro-1-fluoroéthane.

Les exemples qui suivent sont donnés pour illustrer l'invention, de manière non limitative. L'exemple 1 est donné à titre de référence.

### Exemples

### Exemple 1R (de référence)

Un prémélange pour la préparation de mousses de polyuréthane est préparé selon la composition suivante, en poids :
- 50 polyol aminé ARCOL 3770 de ARCO
- 50 polyol aminé VORANOL RA 640 de DOW
- 1 eau
- 2 surfactant siliconé B 1048 de GOLDSCHMIDT
- 2 N-methyl-morpholine
- 1,5 N,N-diméthyl-cyclo-hexylamine
- 24 1,1-dichloro-1-fluoroéthane
Une quantité prédéterminée de ce mélange est enfermée dans un flacon en verre maintenu à une température constante de 50 °C durant 10 jours.

Un échantillon est alors prélevé et son analyse par chromatographie en phase gazeuse révèle la présence de 256 mg de 1-chloro-1-fluoroéthane par kg de 1,1-dichloro-1-fluoroéthane.

### Exemple 2

Dans un prémélange identique à celui de l'exemple 1R, on ajoute avant vieillissement 0,5 % en poids de chlorure de calcium anhydre par rapport au poids de 1,1-dichloro-1-fluoroéthane.

Après vieillissement dans des conditions identiques à celles de l'exemple 1R, on mesure par chromatographie en phase gazeuse seulement 29 mg de 1-chloro-1-fluoroéthane par kg de 1,1-dichloro-1-fluoroéthane.

### Exemple 3

Dans un prémélange identique à celui de l'exemple 1R, on ajoute avant vieillissement 5 % en poids de chlorure de calcium anhydre par rapport au poids de 1,1-dichloro-1-fluoroéthane.

Après vieillissement dans des conditions identiques à celles de l'exemple 1R, la quantité de 1-chloro-1-fluoroéthane présent est inférieure au seuil de détection par chromatographie en phase gazeuse qui est de 5 mg par kg de 1,1-dichloro-1-fluoroéthane.

Par comparaison avec l'exemple 1R de référence, les exemples 2 et 3 illustrent que la quantité de 1-chloro-1-fluoroéthane formé est très nettement inférieure et donc que la stabilité du 1,1-dichloro-1-fluoroéthane est très sensiblement améliorée en présence de chlorure de calcium selon l'invention.

## Revendications

1. Procédé pour la stabilisation de compositions comprenant au moins un hydrocarbure aliphatique halogéné comprenant au moins un atome de fluor, au moins un atome d'hydrogène et, éventuellement un ou plusieurs atomes de chlore, autre que le dichlorotrifluoroéthane, caractérisé en ce qu'on ajoute, par rapport au poids total de l'hydrocarbure aliphatique halogéné compris dans la composition, de 0,05 à 5 % en poids d'au moins un sel d'au moins un métal choisi parmi le lithium et/ou les métaux alcalino-terreux.

2. Procédé selon la revendication 1 pour la stabilisation de compositions essentiellement constituées d'un ou de plusieurs hydrocarbures aliphatiques halogénés.

3. Procédé selon la revendication 1 pour la stabilisation de compositions comprenant du 1,1-dichloro-1-fluoroéthane.

4. Procédé selon la revendication 1, caractérisé en outre en ce qu'un métal alcalino-terreux est le calcium.

5. Procédé selon la revendication 1, caractérisé en outre en ce qu'au moins un sel d'au moins un métal choisi parmi le lithium et/ou les métaux alcalino-terreux est un chlorure.

6. Procédé selon la revendication 1, caractérisé en outre en ce qu'on ajoute de 0,1 à 2 % en poids de sel d'au moins un métal choisi parmi le lithium et/ou les métaux alcalino-terreux par rapport au poids total de l'hydrocarbure aliphatique halogéné compris dans la composition.

7. Compositions comprenant au moins un hydrocarbure aliphatique halogéné comprenant au moins un atome de fluor, au moins un atome d'hydrogène et, éventuellement un ou plusieurs atomes de chlore, autre que le dichlorotrifluoroéthane, caractérisées en ce qu'elles comprennent, par rapport au poids total de l'hydrocarbure aliphatique halogéné compris dans la composition, de 0,05 à 5 % en poids d'au moins un sel d'au moins un métal choisi parmi le lithium et/ou les métaux alcalino-terreux.

8. Compositions selon la revendication 7 constituées de prémélanges, comprenant des hydrocarbures aliphatiques halogénés comme agents gonflants, destinés à la fabrication de mousses de polyuréthane.

9. Compositions selon la revendication 8 constituées de prémélanges à base de polyols, comprenant des hydrocarbures aliphatiques halogénés comme agents gonflants, destinés à la fabrication de mousses de polyuréthane.

10. Compositions selon la revendication 9 constituées de prémélanges à base de polyols, comprenant du 1,1-dichloro-1-fluoroéthane comme agent gonflant, destinés à la fabrication de mousses de polyuréthane.

## Claims

1. Process for the stabilization of compositions containing at least one halogenated aliphatic hydrocarbon containing at least one fluorine atom, at least one hydrogen atom and, optionally, one or more chlorine atoms, other than dichlorotrifluoroethane, characterized in that there is added, with respect to the total weight of the halogenated aliphatic hydrocarbon contained in the composition, from 0.05 to 5% by weight of at least one salt of at least one metal chosen from lithium and/or the alkaline-earth metals.

2. Process according to Claim 1 for the stabilization of compositions composed essentially of one or more halogenated aliphatic hydrocarbons.

3. Process according to Claim 1 for the stabilization of compositions containing 1,1-dichloro-1-fluoroethane.

4. Process according to Claim 1, additionally characterized in that an alkaline-earth metal is calcium.

5. Process according to Claim 1, additionally characterized in that at least one salt of at least one metal chosen from lithium and/or the alkaline-earth metals is a chloride.

6. Process according to Claim 1, additionally characterized in that there is added from 0.1 to 2% by weight of salt of at least one metal chosen from lithium and/or the alkaline-earth metals with respect to the total weight of the halogenated aliphatic hydrocarbon contained in the composition.

7. Compositions containing at least one halogenated aliphatic hydrocarbon containing at least one fluorine atom, at least one hydrogen atom and, optionally, one or more chlorine atoms, other than dichlorotrifluoroethane, characterized in that they contain, with respect to the total weight of the halogenated aliphatic hydrocarbon contained in the composition, from 0.05 to 5% by weight of at least one salt of at least one metal chosen from lithium and/or the alkaline-earth metals.

8. Compositions according to Claim 7 composed of premixes, containing halogenated aliphatic hydrocarbons as blowing agents, intended for the manufacture of polyurethane foams.

9. Compositions according to Claim 8 composed of polyol-based premixes, containing halogenated aliphatic hydrocarbons as blowing agents, intended for the manufacture of polyurethane foams.

10. Compositions according to Claim 9 composed of polyol-based premixes, containing 1,1-dichloro-1-fluoroethane as a blowing agent, intended for the manufacture of polyurethane foams.

## Patentansprüche

1. Verfahren zur Stabilisierung Von Zusammensetzungen, die wenigstens einen halogenierten aliphatischen, von Dichlortrifluorethan verschiedenen Kohlenwasserstoff umfassen, der wenigstens ein Fluoratom, wenigstens ein Wasserstoffatom und gegebenenfalls ein oder mehrere Chloratome umfaßt, dadurch gekennzeichnet, daß man, bezogen auf das Gesamtgewicht des halogenierten aliphatischen Kohlenwasserstoffs in der Zusammensetzung, 0,05 bis 5 Gewichts-% wenigstens eines Salzes wenigstens eines Metalls, das unter Lithium und/oder den Erdalkalimetallen ausgewählt ist, zusetzt.

2. Verfahren gemäß Anspruch 1 zur Stabilisierung von Zusammensetzungen, die im wesentlichen aus einem oder mehreren halogenierten aliphatischen Kohlenwasserstoffen bestehen.

3. Verfahren gemäß Anspruch 1 zur Stabilisierung von Zusammensetzungen, die 1,1-Dichlor-1-fluorethan umfassen.

4. Verfahren gemäß Anspruch 1, außerdem dadurch gekennzeichnet, daß ein Erdalkalimetall Calcium ist.

5. Verfahren gemäß Anspruch 1, außerdem dadurch gekennzeichnet, daß wenigstens ein Salz wenigstens eines Metalls, das unter Lithium und/oder den Erdalkalimetallen ausgewählt ist, ein Chlorid ist.

6. Verfahren gemäß Anspruch 1, außerdem dadurch gekennzeichnet, daß man 0,1 bis 2 Gewichts-% Salz wenigstens eines Metalls, das unter Lithium und/oder den Erdalkalimetallen ausgewählt ist, bezogen auf das Gesamtgewicht des halogenierten aliphatischen Kohlenwasserstoffs in der Zusammensetzung, zusetzt.

7. Zusammensetzungen, die wenigstens einen halogenierten aliphatischen, von Dichlortrifluorethan verschiedenen Kohlenwasserstoff umfassen, der wenigstens ein Fluoratom, wenigstens ein Wasserstoffatom und gegebenenfalls ein oder mehrere Chloratome umfasst, dadurch gekennzeichnet, daß sie, bezogen auf das Gesamtgewicht des halogenierten aliphatischen Kohlenwasserstoffs in der Zusammensetzung, 0,05 bis 5 Gewichts-% wenigstens eines Salzes wenigstens eines Metalls, das unter Lithium und/oder den Erdalkalimetallen ausgewählt ist, umfassen.

8. Zusammensetzungen gemäß Anspruch 7, die aus für die Herstellung von Polyurethanschaumstoffen bestimmten Vormischungen, die halogenierte aliphatische Kohlenwasserstoffe als Treibmittel umfassen, bestehen.

9. Zusammensetzungen gemäß Anspruch 8, die aus für die Herstellung von Polyurethanschaumstoffen bestimmten Vormischungen auf der Basis von Polyolen, die halogenierte aliphatische Kohlenwasserstoffe als Treibmittel umfassen, bestehen.

10. Zusammensetzungen gemäß Anspruch 9, die aus für die Herstellung von Polyurethanschaumstoffen bestimmten Vormischungen auf der Basis von Polyolen, die 1,1-Dichlor-1-fluorethan als Treibmittel umfassen, bestehen.
